(19) Europäisches Patentamt

European Patent Office

Office européen des brevets

(11) Veröffentlichungsnummer: **0 209 685**
**A2**

(12) **EUROPÄISCHE PATENTANMELDUNG**

(21) Anmeldenummer: 86107559.6

(22) Anmeldetag: 04.06.86

(51) Int. Cl.⁴: **A61B 17/58**

(30) Priorität: 28.10.85 DE 3538238
12.07.85 DE 8520206 U

(43) Veröffentlichungstag der Anmeldung:
28.01.87 Patentblatt 87/05

(84) Benannte Vertragsstaaten:
AT BE CH FR GB IT LI NL SE

(71) Anmelder: fischer-werke Artur Fischer GmbH
& Co. KG
Weinhalde 14-18
D-7244 Tumlingen/Waldachtal 3(DE)

(72) Erfinder: Fischer, Artur, Prof. Dr. h. c.
Weinhalde 34
D-7244 Waldachtal 3/Tumlingen(DE)
Erfinder: Kramer, Wolfgang, Dr, med.
Feldbergstrasse 10
D-7031 Oberjettingen(DE)

(54) **Befestigungselement für die Osteosynthes.**

(57) Die Erfindung betrifft ein Befestigungselement für die Osteosynthese, insbesondere für die Fixation von Knochenfragmenten über eine Abstützplatte. Das Befestigungselement besteht aus einer Dübelhülse und einer in die Dübelhülse eingreifenden Schraube, wobei die Dübelhülse aus einem zähelastischen, gewebeveträglichen Kunststoff ist und ihre Außenfläche ein Gewinde aufweist. Die Dübelhülse ist an ihrem dem Schraubenkopf zugewandten Ende geringfügig und an ihrem anderen Ende in stärkerem Maße aufspreizbar, wodurch eine einfache Montage und eine feste Verspannung im Knochengewebe erzielt wird. In Verbindung mit einem metallischen Schraubendreher kann die Position der Dübelhülse bei deren Montage am Röntgenschirm oder in Verbindung mit einer Meßskala ständig überwacht werden.

EP 0 209 685 A2

## Fig.1

## Befestigungselement für die Osteosynthese

Die Erfindung betrifft ein Befestigungselement für die Osteosynthese, insbesondere für die Fixation von Knochenfragmenten über eine Abstützplatte.

Je nach Art der Knochenfraktur werden zur Fixation der Knochenfragmente Zugschrauben unmittelbar oder in Verbindung mit Abstützplatten verwendet. In Abhängigkeit vom Einsatzort werden Kortikalis-oder Spongiosaschrauben verwendet, die sich in ihrer Gewindeform unterscheiden. Die Spongiosaschrauben weisen ein sehr ausgeprägtes tiefes Gewinde auf, um in dem sehr weichen Knochengewebe einen ausreichenden Halt zu erzielen. Nachteilig bei diesen Schrauben ist allerdings, daß sie nach der Heilung der Fraktur nur sehr schwer insbesondere aus der Kortikalis ausschraubbar sind. Als weiterer Nachteil bei der direkten Knochenverschraubung kommt hinzu, daß die unmittelbare Verbindung zwischen Schraube und Knochen unelastisch ist. Kurzzeitige Überbelastungen des Knochens führen zu Schädigungen der Verbindung, die zu einer Lockerung, gegebenenfalls sogar zu einem Ausbruch der Schrauben führen können.

Der Erfindung liegt die Aufgabe zugrunde, eine Befestigungselement für die Osteosynthese zu - schaffen, das hohe Haltewerte ermöglicht, Überbelastungen elastisch auffangen kann und problemlos montierbar und demontierbar ist.

Erfindungsgemäß wird dies durch die Kombination der Merkmale des Anspruches 1 erreicht.

Für des Setzen des Befestigungselementes wird in den Knochen eine etwa dem Kerndurchmesser der Dübelhülse entsprechende Bohrung eingebracht. Mit einem Gewindeschneider wird in den Knochen ein Gewinde geschnitten, das in der Steigung und im Gewindeprofil etwa dem Gewinde der Dübelhülse entspricht. Danach kann die Dübelhülse mit einem Schraubendreher oder Sechskantschlüssel, der in die innere Profilierung der Dübelhülse eingreift, so weit im Bereich der Fraktur eingedreht werden, daß die Dübelhülse im frakturfernen Bereich zu liegen kommt. Dabei kann es häufig erforderlich sein, daß die Bohrung für die Aufnahme der Dübelhülse die Kortikalis des entferntliegenden Fragments vollständig durchdringen muß, um eine sichere Befestigung zu erhalten. Beim Eindrehen der Schraube in die Dübelhülse wird die Dübelhülse an dem dem Schraubenkopf zugewandten hinteren Ende geringfügig aufgeweitet, so daß sich die Dübelhülse wenigstens so fest verklemmt, daß sie sich beim Eindrehen der Schraube nicht mitdrehen kann. Auf diese Weise wird sicher verhindert , daß die Dübelhülse sich beim Eindrehen der Schraube mitdreht und dadurch ihre Position verändert oder gar an dem dem Schraubenkopf gegenüberliegenden Ende der Bohrung aus dem Knochen wieder austritt.Beim Eindrehen der Schraube schneiden sich die Gewindespitzen der Schraube im hinteren Teil der Innenbohrung leicht in die Innenwandung der aus einem gewebeverträglichen Kunststoff bestehenden Dübelhülse ein und bewirken dabei die genannte Aufweitung. Der vordere Teil der Dübelhülse wird beim weiteren Eindrehen der Schraube wesentlich stärker aufgeweitet, wobei die in diesem Teilbereich befindlichen Gewindegänge der Dübelhülse fest in das Knochengewebe eingedrückt werden, so daß eine hochbelastbare Festsetzung der Dübelhülse erzielt wird. Der Schraubenkopf liegt am kopfnahen Fragment oder an einer zusätzlich verwendeten Abstützplatte an und bewirkt eine Verspannung der benachbarten Fragmente im Bereich der Frakturfläche.

Da die am hinteren Ende der Dübelhülse beim Eindrehen der Schraube erfolgende Aufweitung lediglich dazu dient, ein Weiterdrehen der Dübelhülse zu verhindern, erfolgt hier nur eine geringfügige Aufweitung.

Die unterschiedlich starke Aufweitung in den beiden Endbereichen der Dübelhülse wird dadurch erreicht, daß der Innendurchmesser im hinteren Teilstück deutlich größer als der Innendurchmesser im vorderen Teilstück ist. Die Dübelhülse besitzt vorzugsweise an beiden Enden jeweils zwei gegenüberliegende Längsschlitze, die eine Aufspreizung beim Einführen der Schraube ermöglichen. Am hinteren Bereich kann die Länge des Schlitzes beispielsweise zwischen 1/10 und 1/5 der Länge der Dübelhülse betragen, während im vorderen Teilstück die Längsschlitze beispielsweise bis in die Mitte der Dübelhülse verlaufen.

Nach dem in den Knochenfragmenten die Bohrung und mittels eines Gewindeschneiders das Gewinde für die Dübelhülse eingebracht wurden,kann die Dübelhülse mit einem Schraubendreher in die Bohrung eingeschraubt werden. Zu diesem Zweck besitzt die Dübelhülse am hinteren Teilstück ein Innenprofil für die Aufnahme eines entsprechend profilierten Schraubendrehers, wobei sich das Innenprofil über ein definiertes Teilstück bis zu einem Anschlag erstreckt, an dem die Schraubendreherspitze oder ein am Schraubendreherschaft ausgebildeter Vorsprung oder Anschlag beim Eindrehen der Dübelhülse anliegt. Auf diese Weise erhält der Schraubendreher eine definierte Position in der Dübelhülse, so daß eine am Schraubendreherschaft angebrachte Längenmeßskala exakt den Abstand zu dem genannten Anschlag oder bei entsprechender Skalenanpassung den Abstand zum vorderen Ende der Dübelhülse anzeigt. Die Eindringtiefe der Dübelhülse kann auf diese Weise beim Eindrehen an der genannten Längenmeßskala abgelesen werden. Darüber hinaus besteht die Möglichkeit, das Einsetzen der Dübelhülse mittels eines Röntgengerätes zu verfolgen, da die Position der Schraubendreherspitze verfolgt werden kann. Durch die definierte Lage der Schraubendreherspitze innerhalb der Dübelhülse kann auf die entsprechend exakte Position der Dübelhülse geschlossen werden.

Der Schraubendreherschaft kann im vorderen Bereich an die gesamte innere Profilierung der Dübelhülse angepaßt sein, so daß die Schraubendreherspitze beim Eindrehen der Dübelhülse exakt am vorderen Ende der Dübelhülse liegt.

Um am Bildschirm eines Röntgengerätes die exakte Lage der Dübelhülse verfolgen zu können, kann die Dübelhülse auch wenigstens teilweise metallisiert sein.

Die Erfindung wird nachfolgend anhand der Zeichnung, die ein Ausführungsbeispiel zeigt, näher erläutert. Es zeigen:

Figur 1 eine erfindungsgemäße Dübelhülse im Schnitt mit Schraube,

Figur 2 einen Schraubendreher zum Eindrehen der in Figur 1 dargestellten Dübelhülse und

Figur 3 das im Knochen verankerte Befestigungselement bestehend aus Dübelhülse und Schraube.

Das Befestigungselement besteht aus einer Edelstahlschraube 1 und einer Dübelhülse 2, die aus einem gewebeverträglichen Kunststoff, beispielsweise einem ultrahochmolekularen Polyäthylen hergestellt ist. Das hintere Teilstück 3a der Innenbohrung 3 weist einen Durchmesser auf, der etwas geringer ist als der Gewindeaußendurchmesser der Schraube 1. Damit schneiden sich die Gewindespitzen beim Eindrehen der Schraube 1 geringfügig in die Innenwandung der Dübelhülse 2 ein, wobei gleichzeitig im Bereich des hinteren Teilstücks 2a der Dübelhülse 2 eine geringfügige Aufweitung im Sinne einer Aufspreizung erfolgt. Die Dübelhülse 2 besitzt zu diesem Zweck im Bereich des hinteren Teilstücks 2a zwei gegenüberliegende Längsschlitze 4, die eine Aufweitung im Sinne einer Aufspreizung der Dübelhülse 2 zulassen. Ferner ist das Teilstück 3a der Innenbohrung 3 mit einem Innensechskant 5 versehen, der zum Ansetzen eines Sechskantschlüssels oder eines entsprechend profilierten Schraubendrehers dient. Nach dem Teilstück 3a verjüngt sich die Innenbohrung 3 auf einen Durchmesser, der kleiner ist als der Kerndurchmesser der Schraube 1. Im Bereich des vorderen Teilstücks 2b der Dübelhülse 2 weist diese zwei gegenüberliegende Längsschlitze 6 auf,die sich etwa über die Hälfte der Länge der Dübelhülse 2 erstrecken, so daß dieser Bereich beim Eindrehen der Schraube 1 aufgespreizt wird. Da die Längsschlitze 6 deutlich länger sind als die Längsschlitze 4 und da der Innendurchmesser im Bereich der Längsschlitze 6 deutlich kleiner ist als im Bereich der Längsschlitze 4,erfolgt beim Eindrehen der Schraube 1 in die Dübelhülse 2 eine deutlich stärkere Aufweitung am vorderen Teilstück 2b als am hinteren Teilstück 2a. Die Aufweitung bzw. Aufspreizung am hinteren Teilstück 2a dient zur Sicherung der Dübelhülse 2 gegen ein unerwünschtes Verdrehen beim Eindrehen der Schraube 1, während die Aufweitung am vorderen Teilstück 2b die eigentliche Verspannung der Dübelhülse 2 bewirkt, so daß die Dübelhülse 2 auch bei einem starken Anziehen der Schraube 1 und bei dadurch bedingten hohen Zugkraften dennoch sicher im Knochen gehalten wird.

Die Außenfläche der Dübelhülse 2 ist über ihre gesamte Länge mit einem Gewinde 7 versehen, dessen Gewindespitzen und Gewindetäler gerundet sind. Die Dübelhülse 2 kann einen hier nicht dargestellten gewindefreien Teilbereich haben, auf dem eine Metallisierung aufge bracht ist, die eine Erfassung der Dübelhülse im Röntgenbild ermöglicht.

Zum Setzen des Befestigungselementes wird zunächst ein etwa dem Kerndurchmesser der Dübelhülse 2 entsprechendes Bohrloch gebohrt. Danach wird mit einem Gewindeschneider im Knochengewebe ein Gewinde erstellt, das dem Gewinde 7 der Dübelhülse 2 entspricht. Um die Zerstörung des Knochengewebes so gering wie möglich zu halten, wird ein Gewindeschneider mit einem vorderen, schneidenden und einem daran anschließenden gewindeformenden Abschnitt verwendet. Ein derartiger Gewindeschneider ist in der deutschen Patentanmeldung P 35 24 946.3 beschrieben.

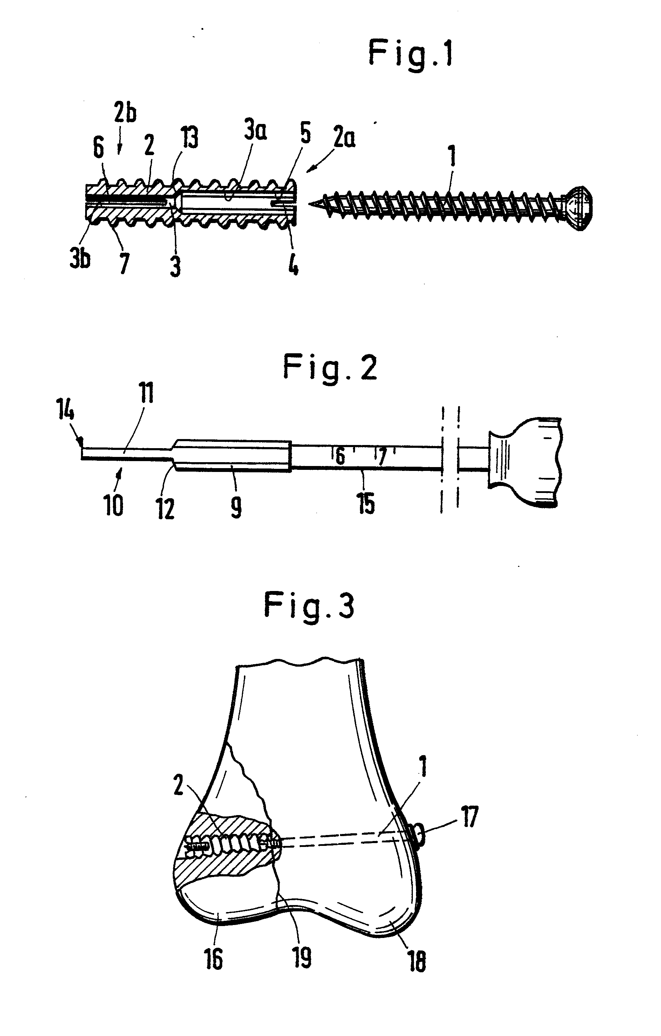

Mittels des in Figur 2 dargestellten Schraubendrehers kann die Dübelhülse 2 in eine Gewindebohrung 8, wie sie in Figur 3 dargestellt ist,eingeschraubt werden. Der Schraubendreher besitzt für diesen Zweck ein Sechskantprofil 9, welches dem Innensechskant 5 der Dübelhülse 2 entspricht. Am vorderen Ende 10 verjüngt sich der Schraubendreher zu einem stabförmigen Endstück 11, dessen Durchmesser an den Innendurchmesser der Dübelhülse 2 in deren vordem Teilstück 2b angepaßt ist. Der Schraubendreher läßt sich nun so weit in die Dübelhülse 2 einsetzen, bis das Übergangsstück 12, welches das Sechskantprofil 9 begrenzt, an einem entsprechenden Anschlag 13 in der Dübelhülse 2 anliegt. Die Spitze 14 des Schraubendrehers liegt dann in der selben Ebene wie das vordere Ende der Dübelhülse 2. Da der Schraubendreher aus metallischem Material besteht, kann beim Eindrehen der Dübelhülse 2 deren Position ständig am Röntgenschirm verfolgt werden, wobei die Spitze 14 des Schraubendrehers gleichzeitig die Lage des vorderen Endes der Dübelhülse 2 anzeigt. Außerdem besteht die Möglichkeit, am Schaft 15 des Schraubendrehers eine Meßskala vorzusehen, an der die jeweilige Eindringtiefe der Dübelhülse 2 ablesbar ist.

Der Schraubendreher kann auch ohne das stabförmige Teil 11 gefertigt sein, wobei die Meßskala dennoch so auf dem Schaft 15 angeordnet sein kann, daß diese Längenmeßskala den Abstand zum vorderen Ende der zugehörigen Dübelhülse angibt.

In Figur 3 ist die Lage der Dübelhülse 2 in einem frakturfernen Fragment 16 dargestellt, während der Schraubenkopf 17 am frakturnahen Fragment 18 anliegt. Die Dübelhülse 2 befindet sich in einer durchgehenden Bohrung und wird insbesondere durch den Aufspreizdruck im vorderen Teilstück 2b sicher im Fragment 16 verankert. Durch die Verwendung einer derartigen Dübelhülse läßt sich ein hoher Anpressdruck im Bereich der Frakturfläche 19 erzielen.

Nach Heilung der Fraktur wird die Schraube 1 wieder herausgedreht, so daß wiederum mit einem entsprechenden Schraubendreher bzw. Sechskantschlüssel die Dübelhülse 2 aus der Gewindebohrung im Knochen ausgedreht werden kann.

**Ansprüche**

1. Befestigungselement für die Osteosynthese, insbesondere für die Fixation von Knochenfragmenten, dadurch gekennzeichnet, daß das Befestigungselement aus einer Dübelhülse (2) und einer in diese eingreifenden Schraube (1) besteht, daß die Außenfläche der Dübelhülse (2) gewindeförmig ausgebildet ist, und daß die Dübelhülse (2) an ihrem vorderen und an ihrem hinteren, dem Schraubenkopf (17) zugewandten Teilstück aufweitbar ist.

2. Befestigungselement nach Anspruch 1, dadurch gekennzeichnet, daß die Dübelhülse (2) an ihrem hinteren Teilstück (2a) in geringerem Maße aufweitbar ist als an ihrem vorderen Teilstück (2b).

3. Befestigungselement nach einem der Ansprüche 1 oder 2, dadurch gekennzeichnet, daß die Dübelhülse (2) im vorderen Teilstück (2b) eine im Durchmesser kleinere Innenbohrung (6) als im hinteren Teilstück (2a) hat.

4. Befestigungselement nach einem der vorhergehenden Ansprüche, dadurch gekennzeichnet, daß das hintere Teilstück (3a) der Innenbohrung - (3)

im Durchmesser geringfügig kleiner als der Gewindeaußendurchmesser der Schraube (1) und der Durchmesser des vorderen Teilstücks (3b) der Innenbohrung (3) kleiner als der Kerndurchmesser der Schraube (1) ist.

5. Befestigungselement nach einem der vorhergehenden Ansprüche, dadurch gekennzeichnet, daß die Dübelhülse (2) vom vorderen Ende ausgehend wenigstens einen über einen Teil ihrer Länge sich erstreckenden ersten Längsschlitz (6) und von ihrem hinteren Ende ausgehend wenigstens einen über einen Teil ihrer Länge sich erstreckenden zweiten, kürzeren Längsschlitz (4) aufweist.

6. Befestigungselement nach einem der vorhergehenden Ansprüche, dadurch gekennzeichnet, daß die Dübelhülse (2) vom hinteren Ende ausgehend ein Innenprofil für die Aufnahme eines entsprechend profilierten Schraubendrehers hat, und daß sich das Innenprofil über ein definiertes Teilstück bis zu einem Anschlag (13) erstreckt, an dem die Schraubendreherspitze oder ein am Schraubendreherschaft (15) ausgebildeter Vorsprung oder Anschlag (12) beim Eindrehen der Dübelhülse (2) anliegt.

7. Befestigungselement nach Anspruch 6, dadurch gekennzeichnet, daß die Schraubendreherspitze (14) exakt bis zum vorderen Ende der Dübelhülse (2) in diese einschiebbar ist.

8. Befestigungselement nach einem der Ansprüche 6 oder 7, dadurch gekennzeichnet, daß der Schraubendreherschaft (15) eine Längenmeßskala trägt, die den Abstand zur Schraubendreherspitze (14) oder bis zum vorderen Ende der Dübelhülse (2) angibt.

9. Befestigungselement nach einem der Ansprüche 6 bis 8, dadurch geennzeichnet, daß das Innenprofil ein Innensechskant (5) ist.

10. Befestigungselement nach einem der vorhergehenden Ansprüche, dadurch gekennzeichnet, daß die Dübelhülse (2) aus einem zähelastischen, gewebeverträglichen Kunststoff besteht.

11. Befestigungselement nach einem der vorhergehenden Ansprüche, dadurch gekennzeichnet, daß die Dübelhülse (2) wenigstens teilweise metallisiert ist.

# Fig.1

# Fig.2

# Fig.3